# EUROPEAN PATENT APPLICATION

(11) **EP 1 803 457 A1**
(43) Date of publication of application: **04.07.2007**
(21) Application number: 05113112.6
(22) Date of filing: 30.12.2005
(51) Int. Cl.: A61K 31/47

(54) **Pharmaceutical composition containing montelukast**

(71) Applicant: Krka Tovarna Zdravil, D.D., Novo Mesto, 8501 Novo Mesto (SI)
(72) Inventor: Kroselj, Vesna, 8310 Sentjernej (SI); Jakse, Renata, 8220 Smarjeske Toplice (SI); Urska Turk, 8000 Novo mesto (SI); Franci Bevec, 8310 Sentjernej (SI)
(74) Representative: UEXKÜLL & STOLBERG

(57) **Abstract**

Pharmaceutical composition comprising montelukast or pharmaceutically acceptable salts thereof as the active ingredient and a method for producing the same. The composition is characterized in that the active ingredient is present in the form of particles having a d₉₀ value of less than 250 µm. The invention also pertains to tablets comprising montelukast which are prepared by direct compression.

## Description

The present invention relates to a pharmaceutical composition containing montelukast or an alkali salt thereof as the active ingredient.

### Background of the invention

Montelukast (INN) is described chemically as [R-(E)]-1-[[[1-[3-[2-[7-chloro-2-quinolinyl]ethenyl]phenyl]-3-[2-(1-hydroxy-1-methylethyl)phenyl]propyl]thio]methyl]cyclopropaneacetic acid. Its monosodium salt is represented by the formula:

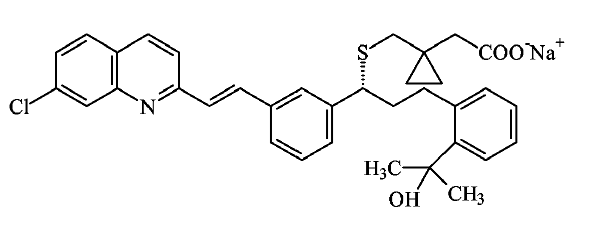

The empirical formula is C₃₅H₃₅ClNNaO₃S, and its molecular weight is 608.18. Montelukast sodium is a hygroscopic, optically active, white to off-white powder. Montelukast sodium is freely soluble in ethanol, methanol, and water and practically insoluble in acetonitrile.

WO 2004/108679 discloses a method for the preparation of montelukast acid and its sodium salt thereof in amorphous form.

Montelukast sodium is a selective and orally active leu-cotriene receptor antagonist that inhibits the cysteinyl leu-cotriene CysLT₁ receptor. It is active as anti-asthmatic, anti-allergic, anti-inflammatory and cryoprotective agent and is hence useful in the treatment of angina, cerebral spasm, glomerular nephritis, hepatitis, endotoxemia, uveitis, and allograft rejection.

Montelukast sodium is marketed in the form of film coated tablets, chewing tablets and granules under the trade name SINGU-LAIR^{®}. The commercially available film coated SINGULAIR^{®} tablets contain microcrystalline cellulose, lactose monohydrate, croscarmellose sodium, hydroxypropyl cellulose, magnesium stearate and coating which comprises hyproxypropyl methylcellulose, hydroxypropyl cellulose, titanium dioxide, red and yellow ferric oxide and carnauba wax.

WO 03/035036 discloses pharmaceutical formulations of montelukast sodium in the form of a granular powder. The granules are prepared by coating a suitable substrate such as mannitol with an aqueous solution of montelukast sodium and drying the coated drug granules. The dried granules are blended with a pharmaceutically acceptable lubricant to produce a flowable and dispersible composition suitable for packaging.

WO 2004/091585 discloses orally disintegrating tablets, containing silicified microcrystalline cellulose. The tablets may be made by dry granulation, wet granulation, or direct compression. While the active agent of the tablets may be subjected to a suitable pre-treatment such as coating, the final tablets are not suitable for further film coating.

CN 1628666 discloses a dispersive tablet of montelukast sodium, which comprises montelukast sodium as main constituent as well as a disintegrating agent, and auxiliary additive. The disintegrating agent and the auxiliary agent are of dispersion tablet type commonly used. The preparation are said to be easily administerable to elderly patients and children. Furthermore, the preparation are said to be stable and quickly absorbable, to have high biological availability and less adverse effects. Such tablets also are not suitable for further film coating.

Wet granulation is a preferred procedure in the manufacture of compressed tablets. However, wet granulation of highly water soluble active ingredients such as montelukast sodium is associated with difficulties caused by a significant increase in granulation viscosity and poor flow properties resulting from the formation of a gel. This problem can be solved by increasing the ultimate tablet size or reducing the drug/excipients ratio to obtain a mixture suitable for agglomeration. Granulation with organic solvents is also possible but is less preferred for environmental reasons and because specially equipped production facilities are needed.

WO 97/16173 suggests the use of a solubility modulator in the manufacture of tablets comprising a large dose of a highly soluble compound such montelukast sodium as active ingredient by wet granulation. In this process the solubility of the active ingredient is reduced by the addition of an electrolyte prior to agglomeration.

The development of a technique for direct compression is more challenging than for wet granulation. It is more difficult to achieve homogeneous mixing of a dry blend composition, and segregation and aggregation of components in the dry blend must be avoided in order to achieve the desired homogeneity of the compression mixture. Additionally, the formulation must have all the characteristics of a free-flowing mixture of the dry blend for direct compression in order to obtain tablets with acceptable hardness and friability, which allows further coating. A further problem of tabletting amorphous montelukast sodium by direct compression resides in the fact that this compound is hygroscopic. This may be the reason why tablets comprising montelukast as the active ingredient and which are prepared by direct compression have not yet been described in the prior art.

### Description of the Invention

It is an object of the present invention to provide tablets comprising montelukast or a pharmaceutically acceptable salt thereof as the active ingredient which are stable and which can be prepared without wet granulation. It is a further object of the present invention to provide a process for preparing such tablets.

Another object of the invention is a dry blend containing montelukast or a pharmaceutically acceptable salt thereof with good free flowing properties which is suitable for direct compression and further film coating.

It was found by the present inventors that this object can be achieved by using montelukast in the form of particles having a d₉₀ value of less than 250 µm, more preferably less than 200 µm, most preferably less 150 µm. The dₓ value indicates that a certain percentage X of the particles has a size below a certain limit. A d₉₀ value of less than 250 µm means that 90 % by volume of the particles have a diameter below 250 µm.

Preferably the average particle size is within the range of 1 to 250 µm, preferably 5 to 200 µm, more preferably 10 to 180 µm and most preferably 20 to 150 µm.

The term "average particle size" as used herein refers to the volume mean diameter of particles. The diameter and volume mean diameter can be determined by laser light scattering using e.g. a Malvern-Mastersizer Apparatus MS 2000. Particle sizes are determined by measuring the angular distribution of laser light scattered by a homogeneous suspension of particles. The size distribution is determined from the light scattering data using the theory of light scattering developed by Gustav Mie.

If montelukast having a d₉₀ value of less than 250 µm is mixed with other ingredients such as diluents and disintegrants, the mixture can easily be formed into tablet cores by direct compression thereof without the addition of water or other solvents. The direct compression is fast, involves only one step prior to compression and includes no solvents, so degradation of the active ingredient during the processing is reduced to a large extend. Furthermore, it was surprisingly found that tablets prepared by direct compression had a significant higher storage stability than tablets prepared by direct compression. Such tablets are also within the scope of the present invention.

In addition to montelukast, the montelukast particles may contain further additives but it is preferred that they essentially and more preferably completely consists of montelukast or pharmaceutically acceptable a salt thereof.

According to the present invention montelukast can be in its acid form or any of its pharmaceutically acceptable salts. It can be used in any polymorphic or pseudoploymorphic form, e.g. in the form of a solvate or hydrate or preferably in amorphous form.

Preferably, montelukast is used in the form of an alkali metal salt, more preferably in the form of its sodium salt and most preferably the amorphous sodium salt is used.

Preferably montelukast used in the present invention contains less than 5 % by weight of water, more preferably less than 3 % by weight, most preferably less than 2 %. The water content can be determined by Karl Fischer titration. The lower limit for the water content is preferably 0 % by weight as determined by Karl Fischer titration.

The formulation comprises an effective amount of montelukast and/or a salt thereof. The compositions preferably comprise 2 to 20 mg of montelukast in the form of the free acid or an equivalent amount of a salt or solvate. More preferably, the formulations according to the invention comprise 10 mg of montelukast in the form of the free acid or an equivalent amount of a salts or solvate.

The pharmaceutical compositions of the present invention preferably comprise at least one pharmaceutically acceptable excipient. Pharmaceutically acceptable excipients can be selected form the group of diluents, surface active agents, antioxidants, chelating agents, disintegrants, binders, lubricants, glidants etc. Compositions comprising at least one diluent, disintegrant, glidant, lubricant, surface active agent, antioxidant, chelating agent, and/or binder are preferred.

Preferred diluents are selected from anhydrous lactose, lactose monohydrate, modified lactose, such as spray dried lactose, dibasic calcium phosphate, tribasic calcium phosphate, microcrystalline cellulose, silicified microcrystalline cellulose, powdered cellulose, spray dried mixtures of lactose and microcrystalline cellulose, starches, such as maize starch and pregelatinized starch, calcium carbonate, sucrose, glucose, dextrates, dextrins, dextrose, fructose, lactitol, mannitol, sorbitol, and/or mixtures of the foregoing.

Silicified microcrystalline cellulose is an intimate mixture of microcrystalline cellulose with silicon dioxide. The preparation of a preferred form of silicified microcrystalline cellulose is disclosed in US 5,585,115. Silicified microcrystalline cellulose is also commercially available from Penwest under the trade name Prosolv^{®}.

Spray dried mixtures of lactose and microcrystalline cellulose are commercially available from Meggle under the trade name Cellactose^{®}.

Especially preferred diluents are a spray dried mixture of lactose and microcrystalline cellulose, optionally combined with microcrystalline cellulose and/or pregelatinized starch.

The diluent are preferably used in a concentration range of 20% to 95% by weight, more preferably 30% to 80% by weight, even more preferably 40% to 70% by weight and most preferably 50% to 60% by weight.

It is preferred that the pharmaceutical composition of the invention comprises at least one diluent. It is furthermore preferred to use diluents of direct compression grades.

Disintegrants have a major role in the disintegration and the dissolution process of tablets. To ensure a high dissolution rate, the choice of a suitable type and an optimal amount of disintegrant should be taken into consideration. It is preferred that the pharmaceutical composition of the invention comprises at least one disintegrant. Preferred disintegrants include crosslinked carboxymethyl cellulose sodium (croscarmellose sodium), crosslinked polyvinylpyrolidone (crospovidone), sodium starch glycolate, corn starch, potato starch, maize starch and modified starches calcium silicates, low substituted hydroxypropylcellulose and mixtures thereof. Most preferred is crosslinked carboxymethyl cellulose sodium (croscarmellose sodium).

The concentration of the disintegrant is preferably in the range of 0.5% to 15% by weight, particularly from about 1% to 10% by weight, and most preferably 2% to 6% by weight.

The compositions of the present invention preferably also contain a glidant. Glidants are preferably selected from the group consisting of silicon dioxide, e.g. colloidal silica, precipitated silica and pyrogenic silica, talc and aluminium silicate. Pyrogenic silica and in particular pyrogenic silica having a BET surface area of 200 ± 25 m²/g and an average primary particle size of 12 nm, such as AEROSIL^{®} 200, are the most preferred glidants.

As a further component the compositions of the present invention preferably also contain a lubricant. Lubricants are preferably selected from the group consisted of magnesium stearate, magnesium lauryl sulfate, sodium stearyl fumarate, sucrose esters or fatty acid, polyethylene glycol, stearic acid and mixtures thereof. Magnesium stearate is the most preferred lubricant.

The lubricant and glidant are preferably used in a concentration of from 0.1% to 1.5% by weight each.

Optionally, the composition may further comprise at least one pharmaceutically acceptable surface active agent. Preferred surface active agents include emulsifying agents such as those commonly known to one skilled in the art. The surface active agent is preferably selected from the group consisting of sodium lauryl sulfate, glyceryl esters, polyoxyethylene glycol esters, polyoxyethylene glycol ethers, polyoxyethylene sorbitan fatty acid esters, sulphate containing surfactants, polyoxyethylene/polyoxypropylene copolymers and mixtures thereof. The most preferred surfactant is sodium lauryl sulfate.

Optionally, the composition may further comprise at least one pharmaceutically acceptable antioxidant. Preferably the antioxidant is selected from the group consisting of vitamin E acetate, α-tocopherol, ascorbyl palmitate, butylated hydroxyanisole (BHA), butylated hydroxytoluene (BHT), propyl gallate, dithiotreitol, tocopherol polyethyleneglycol succinate (TPGS) and mixtures thereof. Chelating agents can also be used as antioxidants, for example EDTA or cyclodextrins. These chelating agents can be used as the sole antioxidant or in admixture with another antioxidant as defined above.

Optionally, the composition may further comprise at least one pharmaceutically acceptable binding agent. Binders are preferably selected from polyvinylpyrolidone, starches, such as pregellatinized starch or plain starch, cellulose derivatives, such as hydroxypropylmethylcellulose (HPMC), hydroxypropylcellulose (HPC) and carboxymethylcellulose (CMC) and their salts, gelatine and mixtures thereof. The most preferred binder is HPMC.

In addition to the above, the compositions of the present invention can contain further additives such as sweeteners, flavourings, colouring agents, opacifying agents, pigments, mixtures thereof and other suitable compounds known to the skilled person.

The pharmaceutical composition of the present invention preferably comprise:
(i) 1 to 10 % by weight of the active ingredient;
(ii) 20 to 95 % by weight diluent;
(iii) 0.5 to 15 % by weight disintegrant;
(iv) 0.1 to 1.5 % by weight lubricant.

Preferably the excipients used in the pharmaceutical composition of the present invention, in particular the diluent and the disintegrant, have a particle size of 1 to 500 µm, more preferably less than 250 µm and most preferably 10 to 250 µm. It is particularly preferred that all particulate excipients have a particle size within these ranges. It was found that the use of excipients and montelukast having particle sizes as defined above results in high homogeneity and uniformity upon dry blending. Particularly high homogeneity and uniformity are achieved if at least 90 % by weight of the dry blend has a particle size of less than 250 µm. It was also found that the flowability of the dry blend is optimal for preparing tablets by direct compression.

The pharmaceutical compositions of the present invention preferably have the form of a tablet, in particular a tablet obtained by direct compression.

Direct compression of the above compositions provides very good content uniformity with respect to the active ingredient. Preferably, the content uniformity is less than about 7.5%, preferably less than about 5% and more preferably less than about 5%. Most preferably the content uniformity is less than about 3%. The lower limit for the content uniformity is preferably zero. The content uniformity is determined by the corresponding USP test (Uniformity of dosage units, General Chapter 905, 2005), where 10 tablets are assayed individually, after which the arithmetic mean and relative standard deviation (RSD) are calculated. The USP criteria lie within 85-115% of the labelled claim, and the RSD is not greater than 6%.

According to a preferred embodiment of the present invention, the tablets are coated, i.e. comprise an outer coating, preferably a film coating. Preferably the tablets are coated with a compound selected from sugars, hydroxypropyl methyl cellulose, hydroxypropyl cellulose, methyl cellulose, ethyl cellulose, polyvinyl alcohol (PVA), sodium carboxymethyl cellulose, coatings based on methacrylic acid and its esters, such as Eu-dragits^{®}, and mixtures thereof. Such coatings allow distinctive colouring and may enhance the aesthetic appeal and stability of the tablets. Furthermore, film coated tablets do not disintegrate in mouth, they disintegrate in the stomach and/or intestine after swallowing.

The tablets are preferably coated with coating compositions based on PVA, such as Opadry^{®} II HP, cellulose derivatives, such as Opadry^{®} 20A or Opadry^{®} White, or on sodium carboxymethyl cellulose, such as Opagloss^{®}.

The coating is preferably applied in an amount of 0.5% to 5 % by weight, more preferably 1% to 4 % by weight, based on the uncoated tablet.

The tablets are preferably prepared by:
(1) dry blending the active ingredient (montelukast or/and an pharmaceutically acceptable salt thereof) with one ore more excipients;
(2) compressing the dry mixture into tablets.

Preferably, in step (1) the active ingredient is first mixed with diluent and other ingredients except for the lubricant and then the lubricant is mixed with the resulting blend. Blending can be carried out in different mixers, known in the art. For example biconic mixers or high shear mixers can be used.

The tablets preferably have a crushing strength in the range of 25 to 170 N, more preferably in the range of 30 to 140 N. The crushing strength can be determined by standard procedures, for example on a Erweka Multicheck tester.

Subsequent to step (2) the tablets are optionally subjected to coating step (3). Preferably the tablets are film coated, e.g. by use of a coating pan.

Preferably, all process steps are carried out in controlled atmosphere, such as low moisture, oxygen, temperature and light protection.

According to a preferred embodiment of the invention the tablets are packed in a packaging which protects the tablets from light, moisture and oxygen. The latter can be achieved by packaging the product under reduced partial pressure of oxygen, relative to air under normal conditions. An atmosphere with reduced oxygen content or reduced oxygen partial pressure may be obtained by the use of reduced pressure, e.g. by creating a partial vacuum in the package by means of a suitable pump or by partial freezing or liquefying the atmosphere, by the use of an inert gas, such as nitrogen or argon, e.g. flushing the package with the inert gas, or by the use of oxygen absorbents. Preferably the oxygen content of the atmosphere used for manufacturing and packing the tablets as well as in the packaging is less than 10 % by volume, more preferably less than 6 % by volume, and most preferably less than 3 % by volume. The lower limit for the oxygen content is preferably 0 % by volume.

Absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. The examples of commercially available absorbers are Ageless^{™} (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

The packaging is preferably formed of a substantially gas-proof material which prevents gas exchange. The packaging preferably comprises an atmosphere with reduced oxygen partial pressure, relative to air under normal conditions. Preferably the packaging material is selected from the group consisting of aluminium foil, Al-polychloro-3-fluoroethylene homo-polymer/PVC laminates, and bottles. The packaging can preferably have the form of a blister packaging. The invention also provides a packaging for the tablets which is provided with a space for holding an absorbent for trapping and disposal of free oxygen.

The present invention will further be illustrated by means of the following examples.

### Examples

### Example 1

| **Ingredient** | **Amount [mg/tablet]** | **Amount [wt.-%]** |
|---|---|---|
| Montelukast sodium | 10.40* | 5.2 |
| Cellactose 80 | 115.00 | 57.5 |
| Microcrystalline cellulose (Avicel PH 102) | 65.00 | 32.5 |
| Croscarmellose sodium (Ac-di-sol) | 8.00 | 4 |
| Magnesium stearate | 1.60 | 0.8 |
| TOTAL | 200.00 | 100 |

| | | |
|---|---|---|
| * corresponds to 10 mg of montelukast free acid | | |

A 20.0 kg batch was prepared. Montelukast sodium (particle size d₉₀ 125 µm, average 43 µm) and microcrystalline cellulose were mixed to obtain triturate. The obtained triturate was mixed with cellactose 80 and croscarmellose sodium in a high shear mixer. Magnesium stearate was admixed and the obtained mixture was pressed into tablets, using round, slightly biconvex punches to a target hardness of approx. 90 N. The loss on drying of the above compression mixture was 2.3%.

The cores were coated in a coating pan with the following coating:

| **Ingredient** | **Amount [mg/tablet]** |
|---|---|
| Opadry White | 6.00 |
| Iron oxide red | 0.004 |
| Iron oxide yellow | 0.020 |

### Example 2

| **Ingredient** | **Amount [mg/tablet]** | **Amount [wt.-%]** |
|---|---|---|
| Montelukast sodium | 10.40* | 5.2 |
| Cellactose 80 | 141.30 | 70.65 |
| Microcrystalline cellulose (Avicel PH 102) | 40.00 | 20.0 |
| Croscarmellose sodium (Ac-di-sol) | 6.00 | 3.0 |
| Silica, colloidal anhydrous (Aerosil 200) | 0.7 | 0.35 |
| Magnesium stearate | 1.60 | 0.8 |
| TOTAL | 200.00 | 100 |

| | | |
|---|---|---|
| * corresponds to 10 mg of montelukast free acid | | |

A 1 kg batch was prepared. Montelukast sodium (particle size d₉₀ 125 µm, average 43 µm) and a part of cellactose were mixed to obtain triturate. The obtained triturate was mixed with other ingredients, except magnesium stearate in a biconic mixer. Magnesium stearate was admixed and the obtained mixture was pressed into tablets, using round, slightly biconvex punches to a target hardness of approx. 90 N. The loss on drying of the above compression mixture was 1.8%. The cores were coated in a coating pan with the same coating as in example 1.

### Example 3 (Reference example) - Wet Granulation

| **Ingredient** | **Amount [mg/tablet]** | **Amount [%]** |
|---|---|---|
| Montelukast sodium | 10.40* | 5.2 |
| Lactose monohydrate | 89.40 | 44.7 |
| Microcrystalline cellulose (Avicel PH 102) | 85.00 | 42.5 |
| HPC (Klucel EF) | 6.00 | 3 |
| Croscarmellose sodium (Ac-di-sol) | 8.00 | 4 |
| Magnesium stearate | 1.20 | 0.6 |
| Purified water | q.s. (approx. 0.3) | |
| TOTAL | 200.00 | 100 |

| | | |
|---|---|---|
| * corresponds to 10 mg of montelukast free acid | | |

An 800 g batch was prepared. Montelukast sodium (particle size d₉₀ 125 µm, average 43 µm), lactose monohydrate and microcrystalline cellulose were mixed in a granulator and granulated with a solution of Klucel EF. The obtained granulate was dried until the loss on drying of 1.45 % was achieved (measured on Mettler analyzer, 80°C, 20 min). Then, croscarmellose sodium and magnesium stearate were admixed and the obtained mixture was pressed into tablets, using round, slightly biconvex punches to a target hardness of approx. 90 N. The cores were coated in a coating pan with the same coating as in example 1.

### Example 4

| **Ingredient** | **Amount [mg/tablet]** | **Amount [wt.-%]** |
|---|---|---|
| Montelukast sodium | 10.40* | 5.2 |
| Lactose monohydrate (Tablettose) | 134.40 | 67.2 |
| Microcrystalline cellulose (Avicel PH 112) | 45.00 | 22.5 |
| Croscarmellose sodium (Ac-di-sol) | 8.00 | 4.0 |
| Silica, colloidal anhydrous (Aerosil 200) | 0.7 | 0.35 |
| Magnesium stearate | 1.50 | 0.75 |
| TOTAL | 200.00 | 100 |

| | | |
|---|---|---|
| * corresponds to 10 mg of montelukast free acid | | |

A 1 kg batch was prepared. Montelukast sodium (particle size d₉₀ 125 µm, average 43 µm) and a part of lactose monohydrate were mixed to obtain triturate. The obtained triturate was mixed with other ingredients, except magnesium stearate in a biconic mixer. Magnesium stearate was admixed and the obtained mixture was pressed into tablets, using round, and slightly biconvex punches to a target hardness of approx. 90 N. The loss on drying of the above compression mixture was 0.94%. The cores were coated in a coating pan with the same coating as in example 1.

Tablets from above experiments were stored under the following conditions: packed in A1/A1 blister packagings 40°C/75% rel. humidity (6 weeks); open 40°C/75% rel. humidity (6 weeks), packed in glass vials at 40°C and 50°C (4 weeks). The HPLC analysis of the samples was performed and the results were as followed:

| **Reference example -in purities in % area** | | | | | | |
|---|---|---|---|---|---|---|
| **Rt(min)** | | **t=0** | **4 weeks** | | **6 weeks** | |
| | | | **50°C** | **40°C** | **40°C/75% open** | **40°C/75% Alu/alu** |
| 2,2 | | 0,07 | 0,35 | 0,11 | 0,47 | 0,10 |
| 2,6 | | 0,26 | 1,74 | 0,50 | 2,58 | 0,49 |
| 9,6 | | 0,06 | 0,12 | 0,07 | 0,08 | 0,05 |
| 18 | | 0,03 | 0,08 | 0,04 | 0,07 | 0,04 |
| **monte** | | **99,0** | **96,9** | **98,6** | **96,2** | **98,8** |

| **Example 1-in purities in % area** | | | | | | |
|---|---|---|---|---|---|---|
| **Rt(min)** | | **t=0** | **4 weeks** | | **6 weeks** | |
| | | | **50°C** | **40°C** | **40°C/75% open** | **40°C/75%Alu/alu** |
| 2,2 | | 0,07 | 0,13 | 0,07 | 0,45 | 0,07 |
| 2,6 | | 0,23 | 0,63 | 0,30 | 2,6 | 0,28 |
| 9,6 | | 0,05 | 0,11 | 0,06 | 0,05 | 0,03 |
| 18 | | 0,03 | 0,07 | 0,05 | 0,06 | 0,04 |
| **monte** | | **99,1** | **98,2** | **98,9** | **96,3** | **99,0** |

| **Example 2 -impurities in % area** | | | | | | |
|---|---|---|---|---|---|---|
| **Rt(min)** | | **t=0** | **4 weeks** | | **6 weeks** | |
| | | | **50°C** | **40°C** | **40°C/75% open** | **40°C/75%Alu/alu** |
| 2,2 | | 0,07 | 0,13 | 0,08 | 0,56 | 0,07 |
| 2,6 | | 0,23 | 0,62 | 0,49 | 3,5 | 0,28 |
| 9,6 | | 0,05 | 0,10 | 0,05 | 0,06 | 0,04 |
| 18 | | / | 0,06 | 0,04 | 0,06 | 0,04 |
| **monte** | | **99,1** | **98,3** | **99,0** | **95,7** | **99,0** |

The above results clearly show the advantages of direct compression over wet granulation in terms of stability.

### Example 5

| **Ingredient** | **Amount [mg/tablet]** | **Amount [wt.-%]** |
|---|---|---|
| Montelukast sodium | 10.40* | 5.2 |
| Lactose monohydrate (Tablettose) | 142.30 | 71.15 |
| Microcrystalline cellulose (Avicel PH 112) | 45.00 | 22.5 |
| Silica, colloidal anhydrous (Aerosil 200 D) | 0.7 | 0.35 |
| Magnesium stearate | 1.60 | 0.8 |
| TOTAL | 200.00 | 100 |

| | | |
|---|---|---|
| * corresponds to 10 mg of montelukast free acid | | |

A 1 kg batch was prepared. Montelukast sodium and a part of Tablettose were mixed to obtain triturate. The obtained triturate was mixed with other ingredients, except magnesium stearate in a biconic mixer. Magnesium stearate was admixed and the obtained mixture was pressed into tablets, using round, slightly biconvex punches to a target hardness of approx. 90 N. The loss on drying of the above compression mixture was 0.85%. The cores were coated in a coating pan with Opadry II HP (PVA based polymer) - 3% w/w according to the core.

### Example 6

| **Ingredient** | **Amount [mg/tablet]** | **Amount [wt.-%]** |
|---|---|---|
| Montelukast sodium | 10.40* | 5.2 |
| Cellactose 80 | 141.30 | 70.65 |
| Microcrystalline cellulose (Avicel PH 112) | 40.00 | 20.0 |
| Croscarmellose sodium (Ac-di-sol) | 6.00 | 3.0 |
| Silica, colloidal anhydrous (Aerosil 200 D) | 0.7 | 0.35 |
| Magnesium stearate | 1.60 | 0.8 |
| TOTAL | 200.00 | 100 |

| | | |
|---|---|---|
| * corresponds to 10 mg of montelukast free acid | | |

A 1 kg batch was prepared. Montelukast sodium and a part of cellactose were mixed to obtain triturate. The obtained triturate was mixed with other ingredients, except magnesium stearate in a biconic mixer. Magnesium stearate was admixed and the obtained mixture was pressed into tablets, using round, slightly biconvex punches to a target hardness of approx. 90 N. The loss on drying of the above compression mixture was 1.80%. The cores were coated in a coating pan with Opadry II HP (PVA based coating) - 3% w/w according to the core.

### Example 7

| **Ingredient** | **Amount [mg/tablet]** | **Amount [wt.-%]** |
|---|---|---|
| Montelukast sodium | 10.40* | 5.2 |
| Cellactose 80 | 141.30 | 70.65 |
| Microcrystalline cellulose (Avicel PH 112) | 40.00 | 20.0 |
| Croscarmellose sodium (Ac-di-sol) | 6.00 | 3.0 |
| Silica, colloidal anhydrous (Aerosil 200 D) | 0.7 | 0.35 |
| Magnesium stearate | 1.60 | 0.8 |
| TOTAL | 200.00 | 100 |

| | | |
|---|---|---|
| * corresponds to 10 mg of montelukast free acid | | |

A 1 kg batch was prepared. Montelukast sodium and a part of cellactose were mixed to obtain triturate. The obtained triturate was mixed with other ingredients, except magnesium stearate in a biconic mixer. Magnesium stearate was admixed and the obtained mixture was pressed into tablets, using round, slightly biconvex punches to a target hardness of approx. 90 N. The loss on drying of the above compression mixture was 1.80%. The cores were coated in a coating pan with Opadry 20A (coating based on HPC+HPMC) - 3% w/w according to the core.

## Claims

1. Pharmaceutical composition comprising montelukast or a pharmaceutically acceptable salts thereof as the active ingredient, **characterized in that** the active ingredient is present in the form of particles having a d₉₀ value of less than 250 µm.

2. The pharmaceutical composition of claim 1, wherein the particles of active ingredient have a d₉₀ value of less than 200 µm.

3. The composition of any one of the preceding claims, wherein the particles of the active ingredient have a water content of less than 5 % by weight.

4. The composition of any one of the preceding claims, wherein the montelukast is present in form of a alkali metal salt.

5. The composition of claim 4, wherein the montelukast is present in form of its sodium salt.

6. The composition of any one of the preceding claims, wherein the active ingredient is present in amorphous form.

7. The composition of any one of the preceding claims, comprising 2 to 20 mg of the active ingredient.

8. The composition of any one of the preceding claims having the form of a tablet.

9. The composition of any one of the preceding claims, which further comprises a diluent.

10. The composition of claim 9, wherein the diluent is selected from the group consisting of anhydrous lactose, lactose monohydrate, modified lactose, dibasic calcium phosphate, tribasic calcium phosphate, microcrystalline cellulose, silicified microcrystalline cellulose, powdered cellulose, a spray dried mixture of lactose and microcrystalline cellulose, maize starch, pregelatinized starch, calcium carbonate, sucrose, glucose, dextrates, dextrins, dextrose, fructose, lactitol, mannitol, sorbitol, starch, and mixtures thereof.

11. The composition of any one of the preceding claims, which further comprises a disintegrant.

12. The composition of claim 11, wherein the disintegrant is selected from the group consisting of croscarmellose sodium, crospovidone, sodium starch glycolate, corn starch, potato starch, maize starch, modified starches, calcium silicates, low substituted hydroxypropylcellulose, and mixtures thereof.

13. The composition of any one of the preceding claims, which further comprises a glidant.

14. The composition of claim 13, wherein the glidant is selected from the group consisting of silicon dioxide, colloidal silica, precipitated silica, pyrogenic silica, aluminium silicate, and mixtures thereof.

15. The composition of any one of the preceding claims, which further comprises a lubricant.

16. The composition of claim 15, wherein the lubricant is selected from the group consisting of magnesium stearate, magnesium lauryl sulfate, sodium stearyl fumarate, sucrose esters, fatty acids, polyethylene glycol, stearic acid, and mixtures thereof.

17. The composition of any one of the preceding claims, which further comprises a surface active agent.

18. The composition of claim 17, wherein the surface active agent is selected from the group consisting of sodium laurylsulfate, glyceryl esters, polyoxyethylene glycol esters, polyoxyethylene glycol ethers, polyoxyethylene sorbitan fatty acid esters, sulphate containing surfactants, polyoxyethylene/polyoxypropylene copolymers, and mixtures thereof.

19. The composition of any one of the preceding claims, which further comprises an antioxidant.

20. The composition of claim 19, wherein the antioxidant is selected from the group consisting of vitamin E acetate, α-tocopherol, ascorbyl palmitate, butylated hydroxyanisole (BHA), butylated hydroxytoluene (BHT), propyl gallate, dithiotreitol, tocopherol polyethyleneglycol succinate (TPGS), EDTA, cyclodextrins, and mixtures thereof.

21. The composition of any one of the preceding claims, which further comprises a binder.

22. The composition of claim 21, wherein the binder is selected from the group consisting of polyvinylpyrolidone, starch (pregellatinized or plain), cellulose derivatives, hydroxypropylmethylcellulose (HPMC), hydroxypropylcellulose (HPC), carboxymethylcellulose (CMC) and their salts, gelatine, and mixtures thereof.

23. The composition of any one of claims 9 to 22, wherein the diluent, disintegrant and/or binder has a particles size of 1 to 500 µm.

24. The composition of any one of claims 8 to 23, wherein the tablet comprises an outer coating.

25. The composition of claim 24, wherein the outer coating comprises sugar, hydroxypropyl methyl cellulose, hydroxypropyl cellulose, methyl cellulose, ethyl cellulose, polyvinyl alcohol, sodium carboxymethyl cellulose, methacrylic acid and/or methacrylic acid esters.

26. The composition of any of the preceding claims having a crushing strength in the range of 25 to 170 N.

27. The composition of claim 26 having a crushing strength in the range of 30 to 140 N.

28. The composition of any one of claims 1 to 27 comprising:
(i) 1 to 10 % by weight of the active ingredient;
(ii) 20 to 95 % by weight diluent;
(iii) 0.5 to 15 % by weight disintegrant;
(iv) 0.1 to 1.5 % by weight lubricant.

29. A process for the preparation a composition to any one of claims 1 to 28 comprising the steps of
(1) dry blending the active ingredient with one or more excipients,
(2) compressing the dry mixture to tablets.

30. The process of claim 29, further comprising (3) coating of the tablets of step (2).

31. The process of claim 29 or 30, further comprising (4) packing the tablets in a packaging comprising an gas mixture containing less than 10 % by volume of oxygen.

32. The process of any one of claims 29 to 31, wherein the blending, compressing and/or coating is carried out in a controlled atmosphere, including low moisture, low oxygen, low oxygen, low temperature and light protection conditions.

33. Tablet comprising montelukast or a pharmaceutically acceptable salts thereof as the active ingredient and at least one excipient, **characterized in that** the tablet is prepared by direct compression.
